# EUROPEAN PATENT APPLICATION

(11) **EP 1 649 752 A1**
(43) Date of publication of application: **26.04.2006**
(21) Application number: 05256594.2
(22) Date of filing: 25.10.2005
(51) Int. Cl.: A21D 2/18, A21D 2/36, A21D 10/00, A23L 1/09, A23L 1/10, A23L 1/308

(54) **Food additive and high fibre baked food composition**

(30) Priority: 25.10.2004 US 973513
(71) Applicant: THE QUAKER OATS COMPANY, Chicago, Illinois 60661 (US)
(72) Inventor: The Quaker Oats Company, Chicago 60661 IL (US); Lewis, Karen M., Arlington Heights 60004 IL (US)
(74) Representative: Garratt, Peter Douglas

(57) **Abstract**

The present invention is directed to a food additive for making a high fiber, high shelf-stable semi-soft or soft baked food composition. More particularly, the invention relates to a food additive, baked food composition and method for lowering blood cholesterol. A baked food composition of the present invention comprises a grain component and a soluble fiber humectant. Additionally, a baked food composition of the present invention qualifies for a Food and Drug Administration health claim that the baked food composition helps lower cholesterol and may reduce the risk of heart disease.

## Description

The present invention is directed to a food additive for making a high fiber, high shelf-stable semi-soft or soft baked food composition. More particularly, the invention relates to a food additive and a baked food composition. The food additive contains a soluble fiber humectant and an oat or other grain component.

Baked food items and methods for their production are known. Examples of such items include breakfast bars, breads, cookies and cakes. Typical baked food items are high in sugar and/or carbohydrates and low in soluble fiber.
Additionally, typical baked food items do not meet the requirements currently set forth by the Food and Drug Administration (FDA) for a claim that the food item helps lower cholesterol and may reduce the risk of heart disease. Currently, the FDA claim states diets low in saturated fat and cholesterol that include beta-glucan soluble fiber may reduce the risk of heart disease. To qualify for this health claim, a beta-glucan-containing food item must provide at least 0.75 grams of soluble fiber per serving.

Today's consumer desires baked food items that are nutritious, have good texture characteristics, are low in carbohydrates and have a health benefit. Consequently, a need exists for a baked food item that is low in sugar, has a soft or semi-soft texture and qualifies for a health claim that it lowers cholesterol and may reduce the risk of heart disease.

One aspect of the present invention relates to a food additive for making a high fiber, high shelf-stable soft or semi-soft baked food composition. The food additive comprising: a) a soluble fiber humectant consisting of a low molecular weight indigestible maltodextrin; and b) a grain component comprising beta-glucan, wherein, when contacted with water, the grain component binds the water and the soluble fiber humectant binds moisture released by the dehydration of the food additive that occurs during baking. The food additive imparts a soft or semi-soft texture to a baked food composition. This texture can last for extended periods of time, particularly when the baked food composition is stored in a closed container.

Another aspect of the present invention relates to a baked food composition for lowering blood cholesterol in humans. The baked food composition comprising: a) from about 1.5 to about 10.0 wt.% soluble fiber humectant consisting of a low molecular weight indigestible maltodextrin, based on the total weight of the baked food composition; and b) from about 1 to about 60 wt.% of a grain component comprising beta glucan, based on the total weight of the baked food composition, wherein the soluble fiber humectant binds moisture released by the dehydration of the baked food composition that occurs during baking. The binding of water by the humectant imparts softness or semi-softness to the baked food composition. A baked food composition of the present invention qualifies for the FDA health claim that the baked food composition helps lower cholesterol and may reduce the risk of heart disease.

A further aspect of the present invention relates to a method of manufacturing a baked food composition comprising the steps of mixing a soluble fiber humectant consisting of a low molecular weight indigestible maltodextrin with a grain component comprising beta-glucan and with optional other constituents; and baking the mixture such that the soluble fiber humectant binds moisture released by dehydration of the baked food composition that occurs during baking The oat or other grain component binds water so that, during baking, the soluble fiber humectant binds moisture released by the dehydration of the baked food composition that occurs, thereby imparting semi-softness or softness to the baked food composition.

The soluble fiber humectant utilized in the food additive of the present invention and in the baked food composition of the present invention is a low molecular weight indigestible maltodextrin. Preferably, the soluble fiber humectant is a maltodextrin having a total dietary fiber of 85 wt.%, based on the total weight of the maltodextrin, and a viscosity of about 1.5 cp for a 10% solution. Such a soluble fiber humectant is available from Matsutani Chemical Industry Co., Ltd. of Itami-shi Hyogo-ken, Japan under the name FIBERSOL 2®. Preferably, for the food additive of the present invention, the soluble fiber humectant is present in a range of from about 5 to about 15 wt.%, more preferably, about 8 wt.%, based on the total weight of the food additive. Preferably, for the baked food composition of the present invention, the soluble fiber humectant is present in a range of from about 1.5 to about 10.0 wt.%, more preferably, about 3.30 wt.%, based on the total weight of the baked food composition.

The grain component utilized in the food additive of the present invention and in the baked food composition of the present invention can be selected from oat flour, oat bran, oat bran concentrate, defatted oat flour, defatted oat bran, defatted oat bran concentrate, rolled oats, oat crisp rice, crisp oats and mixtures thereof. Preferably, the grain component is oat bran concentrate (available from The Quaker Oats Company, Chicago, IL). More preferably, the grain component is a mixture of oat bran concentrate, oat flour, crisp oats, oat crisp rice and old fashioned rolled oats (such as BUCKEYE ROLLED OATS®, available from The Quaker Oats Company, Chicago, Illinois). Preferably, the grain component comprises at least 2.0 wt.% beta-glucan, based on the total weight of the grain component. Oat bran concentrate, for example, comprises approximately 11.5 wt.% beta-glucan, based on the total weight of the oat bran concentrate.
A sufficient amount of grain component (such as a mixture of oat bran concentrate, oat flour, crisp oats, oat crisp rice and old fashioned rolled oats) is present in the baked food composition to provide a blood cholesterol lowering benefit, preferably a LDL-cholesterol lowering benefit. The sufficient amount of grain component is within the range of from about 1 to about 60 wt.%, preferably, from about 2 to about 45 wt.%, more preferably, from about 30 to about 40 wt.%, based on the total weight of the baked food composition. The cholesterol lowering benefit is provided when a human ingests the baked food composition of the present invention.

When contacted with water, the grain component of the food additive of the present invention and the baked food composition of the present invention binds the water. Preferably, before baking, at least 3.0 wt.% water, based on the total weight of a high fiber, high shelf-stable semi-soft or soft baked food composition, can be present in such a food prepared with the food additive of the present invention. In regard to the baked food composition, preferably, before baking, at least 3.0 wt.% water, based on the total weight of the baked food composition, can be present. "Soft" and "semi-soft" are used herein according to their customary meaning in the art.

Typically, in accordance with the invention, the grain component of the present invention binds sufficient water before baking so that during the dehydration process that occurs during baking, the moisture released is absorbed by the soluble fiber humectant at such a rate so as to impart a moisture content in the baked food composition. Preferably, the soluble fiber humectant imparts a moisture content in the baked food composition of at least 9.0 wt.%, based on the total weight of the baked food composition. A baked food composition having a moisture content, preferably a moisture content of at least 9.0 wt.%, based on the total weight of the baked food composition, remains in a soft or semi-soft state when stored in a closed container. Preferably, the soft or semi-soft texture of the baked food composition of the present invention lasts for three months, more preferably, six months.

Baking the baked food composition typically occurs in an oven. Preferred ovens include direct and indirect heat convection ovens, impingement ovens, microwave ovens and infrared ovens with direct and indirect heat. Convection ovens and impingement ovens are preferred. Indirect heat convection ovens are more preferred. Baking is conducted at temperature in the range of 100°F to 1000°F, preferably in the range of 250°F to 450°F.

The baked food composition of the present invention can contain any edible components typically found in baked food items. Such components can increase or decrease the density of the baked food composition. Examples of such components include viscosity providing agents, bulking agents, flavoring agents, coloring agents, salts, antioxidants, vitamins, minerals, protein sources, sugars, sweeteners, malts, preservatives, leavening agents, fat sources and mixtures thereof.

Examples of viscosity providing agents utilized in the baked food composition of the present invention include soluble and dispersible sweeteners such as sucrose, brown sugar, glucose, fructose and corn syrup; gums; pectin; and starch; high-intensity sweeteners such as aspartame, sucralose and acesulfame potassium; and mixtures thereof. Sweeteners are preferred.

Preservatives utilized in the baked food composition of the present invention include natural and artificial preservatives such as tocopherols, Vitamin C and mixtures thereof. Tocopherols are preferred. Leavening agents utilized in the baked food composition of the present invention include sodium bicarbonate and trisodium phosphate. Sodium bicarbonate is preferred.

Minerals utilized in the baked food composition of the present invention include calcium carbonate. Protein sources utilized in the baked food composition of the present invention include liquid, solid and semi-solid protein sources such as dried whole eggs, milk powder, dairy whey, gelatin, margarine, butter and mixtures thereof. Dried whole eggs are preferred.

Examples of flavoring agents utilized in the baked food composition of the present invention include natural and artificial flavoring agents such as cinnamon, spice oleoresins, vanillin, benzaldehyde, vanilla, almond extract, citrus oils, sugar solutions and mixtures thereof. "Sugar solution" means a solution added to provide additional sweetness and flavor separate from the sugar. The sugar solution is made from sugars (preferably unprocessed), syrups (preferably natural) and mixtures thereof. Examples include honey, maple syrup and mixtures thereof.

Vitamins utilized in the present invention include riboflavin, folio acid, pyridoxine hydrochloride, thiamin, ascorbic acid, cyanocobalamin and mixtures thereof. Binding agents such as sorbitol can be utilized in the food composition of the present invention, as can glycerin. Bulking agents utilized in the baked food composition of the present invention include starch, cellulose fiber, bean fiber and mixtures thereof. Starch is preferred.

The components used to form a baked food composition of the present invention are typically mixed together. Mixing can be accomplished by any manual or automatic mixing method known in the art. Preferably, the components are mixed with a 1200 pound paddle mixer.

After mixing, the components can be fed to an extruder. Any extruder known in the art can be used. Preferably, an APV twin screw extruder is used.
After baking, a baked food composition of the present invention is preferably formed to any desired shape. A square is the preferred shape. Icing can be applied to the top of the baked food composition, either before or after shape formation. The icing can comprise sugar, water, flavoring and a whipping agent. The icing can be applied to the baked food composition by any method known in the art.

While the invention has been described with respect to certain preferred embodiments, as will be appreciated by those skilled in the art, it is to be understood that the invention is capable of numerous changes, modifications and rearrangements and such changes, modifications and rearrangements are intended to be covered by the following claims.

## Claims

1. A food additive for making a high fiber, high shelf-stable semi-soft or soft baked food composition comprising:
a) a soluble fiber humectant consisting of a low molecular weight indigestible maltodextrin; and
b) a grain component comprising beta-glucan;
wherein, when contacted with water, the grain component binds the water and the soluble fiber humectant binds moisture released by the dehydration of the food additive that occurs during baking.

2. The food additive of claim 1 wherein the soluble fiber humectant has a total dietary fiber of 85 wt.%, based on the total weight of the soluble fiber humectant, and a viscosity of about 1.5 cp for a 10% solution.

3. The food additive of claim 1 wherein the grain component is selected from oat flour, oat bran, oat bran concentrate, defatted oat flour, defatted oat bran, defatted oat bran concentrate, rolled oats, oat crisp rice and mixtures thereof.

4. The food additive of claim 1 wherein the grain component comprises at least 2 wt.% beta-glucan, based on the total weight of the grain component.

5. The food additive of claim 1 wherein the soluble fiber humectant is present in a range of from about 5 to about 15 wt.%, based on the total weight of the food additive.

6. The food additive of claim 1 wherein the grain component is present in a range of from about 85 to about 95 wt.%, based on the total weight of the food additive.

7. A baked food composition for lowering blood cholesterol in humans comprising:
a) from about 1.5 to about 10.0 wt.% soluble fiber humectant consisting of a low molecular weight indigestible maltodextrin, based on the total weight of the baked food composition; and
b) from about 1 to about 60 wt.% of a grain component comprising beta-glucan, based on the total weight of the baked food composition;
wherein the soluble fiber humectant binds moisture released by dehydration of the baked food composition that occurs during baking.

8. The baked food composition of claim 7 wherein from about 2 to about 45 wt.% of the grain component is present, based on the total weight of the baked food composition.

9. The baked food composition of claim 7 wherein from about 30 to about 40 wt.% of the grain component is present, based on the total weight of the baked food composition.

10. The baked food composition of claim 7 wherein the baked food composition has a moisture content of at least 9 wt.%, based on the total weight of the baked food composition.

11. The baked food composition of claim 7 wherein the cholesterol is low density lipoprotein-cholesterol.

12. The baked food composition of claim 7 wherein the grain component is selected from oat flour, oat bran, oat bran concentrate, defatted oat flour, defatted oat bran, defatted oat bran concentrate, rolled oats, oat crisp rice and mixtures thereof.

13. The baked food composition of claim 7 wherein the soluble fiber humectant has a total dietary fiber of 85 wt.%, based on the total weight of the soluble fiber humectant, and a viscosity of about 1.5 cp for a 10% solution.

14. The baked food composition of claim 7 wherein the grain component comprises at least 2 wt.% beta-glucan, based on the total weight of the grain component.

15. The baked food composition of claim 7 further comprising flavoring agents, coloring agents, salts, antioxidants, vitamins, minerals, protein sources, sugars, sweeteners, malts, fat sources or mixtures thereof.

16. The baked food composition of claim 7 wherein the baked food composition is soft or semi-soft for at least three months when stored in a closed container.

17. A method of manufacturing a baked food composition comprising the steps of mixing a soluble fiber humectant consisting of a low molecular weight indigestible maltodextrin with a grain component comprising beta-glucan and with optional other constituents; and baking the mixture such that the soluble fiber humectant binds moisture released by dehydration of the baked food composition that occurs during baking.

18. A method according to Claim 17, wherein the mixture before baking has a moisture content of at least 3 wt.%, based on the total weight of the baked food composition.

19. A method according to Claim 17 or Claim 18, wherein the composition contains from about 1.5 to about 10.0 wt.% of said soluble fiber humectant; and from about 1 to about 60 wt.% of said grain component, based on the total weight of the baked food composition.

20. A method according to Claim 19, wherein from about 2 to about 45 wt.% of the grain component is present, based on the total weight of the baked food composition.

21. A method according to Claim 20, wherein from about 30 to about 40 wt.% of the grain component is present, based on the total weight of the baked food composition.

22. A method according to any one of Claims 17 to 21, wherein the baked food composition has a moisture content of at least 9 wt.%, based on the total weight of the baked food composition.

23. A method according to any one of Claims 17 to 22, wherein the grain component is selected from oat flour, oat bran, oat bran concentrate, defatted oat flour, defatted oat bran, defatted oat bran concentrate, rolled oats, oat crisp rice and mixtures thereof.

24. A method according to any one of Claims 17 to 23, wherein the soluble fiber humectant has a total dietary fiber of 85 wt.%, based on the total weight of the soluble fiber humectant, and a viscosity of about 1.5 cp for a 10% solution.

25. A method according to any one of Claims 17 to 24, wherein the grain component comprises at least 2 wt.% beta-glucan, based on the total weight of the grain component.

26. A method according to any one of Claims 17 to 25, wherein said other constituents comprise flavoring agents, coloring agents, salts, antioxidants, vitamins, minerals, protein sources, sugars, sweeteners, malts, fat sources or mixtures thereof.
